Europäisches Patentamt

(19) European Patent Office

Office européen des brevets

(11) Publication number: **0 325 949 B1**

# (12) EUROPEAN PATENT SPECIFICATION

(45) Date of publication of patent specification :
**18.12.91 Bulletin 91/51**

(51) Int. Cl.⁵: **A61K 31/57, A61K 9/08**

(21) Application number: **89100413.7**

(22) Date of filing: **11.01.89**

(54) Concentrated solutions of corticosteroids and method of making them.

(30) Priority: **13.01.88 US 143579**

(43) Date of publication of application:
**02.08.89 Bulletin 89/31**

(45) Publication of the grant of the patent:
**18.12.91 Bulletin 91/51**

(84) Designated Contracting States:
**AT BE CH DE ES FR GB GR IT LI LU NL SE**

(56) References cited:
**EP-A- 0 250 088**
**US-A- 3 900 561**
**US-A- 4 360 518**

(73) Proprietor: **SCHWARZ PHARMA AG**
**Alfred-Nobel-Strasse 10**
**W-4019 Monheim (DE)**

(72) Inventor: **Multhauf, Donna K. c/o Schwarz**
**Parma-Kremers**
**Urban Comp. 5600 West County Line Road**
**Mequon Wisconsin 53092 (US)**
Inventor: **Wagle, Sudhakar S., Dr. c/o Schwarz**
**Parma-Kremers**
**Urban Comp. 5600 West County Line Road**
**Mequon Wisconsin 53092 (US)**

EP 0 325 949 B1

## Description

Numerous compositions comprising corticosteroids are known in the art. These compositions include creams, lotions, gels, emulsions and solutions. In particular, solutions containing up to 1% of a corticosteroid are known.

For example US-A 4,360,518 describes the synergistic effects of certain anti-inflammatory drugs. This patent deals with the art of using nonsteroidal and steroidal drug compositions as effective for treatment for dermatologic disorders.

The basis of the sample formulations presented in this patent is to form a combination product in a single vehicle. The composition of the anti-inflammatory steroidal agent, triamcinolone acetonide never exceeds 0,1% and the established equivalency for hydrocortisone would be 1%. The stabilizing system is composed of an antioxidant and a metal chelating agent. There is no disclosure of solutions containing substantially more than 1% of a corticosteroid and a method of making such solutions using nonionic surfactants.

The percutaneous penetration and therapeutic efficacy of corticosteroids can be increased by increasing their concentration in a given vehicle. Accordingly, solutions containing greater than 1% of a corticosteroid would be more therapeutically effective than the presently known corticosteroid solutions.

It is, therefore, an object of the invention to provide solutions containing substantially more than 1% of a corticosteroid. It is also an object of the invention to provide a method of making these solutions. These and other objects are attained by the present invention as will be apparent from the following description.

In one aspect, the invention comprises a solution of a corticosteriod comprising : at least 2,5% of the corticosteroid ; from at least 25% to 80% of a surfactant ; from 0 to 70% ethanol ; from 0 to 70% propylene glycol ; provided that the amount of ethanol or of propylene glycol or of the two together is at least 15% ; an effective amount of an antimicrobial agent ; and remainder water (all percentages used herein are calculated on a weight : weight basis). Preferred are solutions comprising a nonionic surfactant and those comprising from at least 25% to 45% ethanol, from at least 10% to 30% propylene glycol and from at least 10% to 25% water.

The solutions of the invention are prepared by a method comprising : mixing the surfactant and the corticosteroid with heating until the temperature is from at least 5 to 10°C above the pour point of the surfactant ; separately mixing the remaining ingredients with heating until the temperature is from at least 5 to 10°C above the pour point of the surfactant ; combining the two mixtures ; and heating the combined mixtures at a temperature from at least 5 to 10°C above the pour point of the surfactant with mixing until a solution is formed.

The solutions of the present invention are useful for the topical application of corticosteroids, and they are particularly well-suited for the treatment of hairy areas of the body. However, the solutions of the invention should not be used on sensitive areas of the body such as the eyes.

Due to the substantially increased concentration of corticosteroid that they contain, the solutions of the invention are more therapeutically effective than prior art solutions. Thus, a smaller volume of the solutions of the invention can be used as compared to prior art solutions, or better results will be obtained using equal volumes of the solutions of the invention and of prior art solutions.

Detailed description of the presently preferred embodiments

The term "corticosteroid" is used herein in its usual sense. Particularly preferred in the practice of the invention are the natural and synthetic anti-inflammatory corticosteroids of the 11,17,21-trihydroxypregnane-3,20-dione class and the derivatives of these compounds. This class includes hydrocortisone. The derivatives which are useful in the practice of the invention include the esterified and halogenated derivatives. However, the corticosteroids most advantageously used in the practice of the present invention are those having one or more free alcohol groups since the free alcohol groups contribute to the solubility of the compounds in the solutions of the inventions. Thus, the greatest possible concentration is achieved using the free alcohol form of the corticosteroids. The term "corticosteroid" ist not meant to include the salts of these compounds.

The ethanol which is used in the solutions of the invention can either be 100% ethanol or denatured 95% ethanol. The substance used to denature the ethanol is not critical as long as its presence is not deleterious to the intended uses of the resultant solution.

Although solutions containing ethanol and no propylene glycol and solutions containing propylene glycol and no ethanol can be made, the solutions of the invention preferably contain both compounds. In particular, the solutions of the invention preferably contain from at least 25% to 45% ethanol and from at least 10% to 30% propylene glycol. Corticosteroids, such as hydrocortisone, are generally more soluble in ethanol than in propylene glycol.

However, propylene glycol is less irritating to human skin than is ethanol, and propylene glycol improves the adherence of the solutions to human skin. Thus, the presence of both is desirable.

The solutions of the invention contain from at least 25% to 80% of a surfactant. At a concentration above 80% surfactant, a solution will not form. At a concentration below 25% surfactant, a solution will be formed. However, the resultant solution will not be stable under certain conditions, such as freezing temperatures, that can reasonably be expected during the transport, storage and use of corticosteroids.

Any surfactant can be used to prepare the solutions of the invention as long as it is compatible with the corticosteroid used and with the intended use of the final solution. Since the final solutions will be used for topical applications to human skin and since certain corticosteroids, such as hydrocortisone, decompose above neutral pH, preferred surfactants are nonionic surfactants. Particularly preferred are the nonionic polyoxyethylene surfactants such as Polysorbate 20 (polyoxyethylene (20) sorbitan monolaurate) and ARLASOVE 200® (polyoxyethylene (20) isohexadecyl ether).

Any antimicrobial agent may be used in the solutions of the invention as long as it is compatible with the corticosteroid and with the intended use of the final solution. Preferred are antimicrobial agents which contribute to the solubilizing of the corticosteroid such as benzalkonium chloride and parabens. The antimicrobial agent should, of course, be used at a concentration which is effective to prevent microbial growth. Such concentrations are known in the art.

The water used is deionized water or U.S.P. purified water. It should be noted that the solutions of the invention can be made without using any water. However, the use of water is strongly preferred since solutions containing water are less expensive to make. Also, water is not irritating to human skin. In particular, solutions containing from at least 10 to 25% water are preferred.

To summarize, the most preferred solutions of the invention contain : at least 2,5% of a corticosteroid ; from at least 25% to 55% of a nonionic surfactant ; from 25% to 45% ethanol ; from 10% to 30% propylene glycol ; an effective amount of an antimicrobial agent ; and from 10% to 25% water. These preferred solutions have several desirable properties. In particular, they contain a substantially increased concentration of corticosteroid as compared to prior art solutions ; they have increased therapeutic efficacy as compared to prior art solutions ; they are sufficiently fluid to be easily applied to the skin and to penetrate to the skin on hairy areas of the body ; they are sufficiently viscous so the solutions will adhere to the skin to allow for effective treatment ; and they are nonirritating to most areas of the body.

In the method of the invention the surfactant and the corticosteroid are mixed with heating until the temperature is from 5 to 10°C above the pour point of the surfactant. The corticosteroid does not dissolve in the heated surfactant, and the mixture should be adequately stirred to obtain an even dispersion of the corticosteroid in the surfactant.

The remaining ingredients are separately mixed with heating until the temperature is also from at least 5 to 10°C above the pour point of the surfactant. In heating this mixture, care should be taken to avoid volatilizing the ethanol. Thus, the heating time should be kept to a minimum.

Next, the two mixtures are combined and heated at a temperature from at least 5 to 10°C above the pour point of the surfactant with mixing until all of the corticosteroid is dissolved and a solution is formed. Again, care should be taken not to volatilize the ethanol.

Using the method of the invention, clear, stable solutions containing substantially increased concentrations of corticosteroid as compared to prior art corticosteroid solutions can be obtained. Thus, the solutions of the invention will have greater therapeutic efficacy than prior art solutions.

Example 1

First, 2,5 grams of hydrocortisone was combined with 25,0 grams of Polysorbate 20, and the two ingredients were mixed with heating and stirring until the temperature reached 45°C and an even dispersion of the hydrocortisone in the surfactant was obtained. A second mixture was prepared by combining 35,0 grams of denatured ethanol, 20,5 grams of propylene glycol, 17,0 grams of water and 0,04 grams of benzalkonium chloride and then mixing the combined ingredients with heating until the temperature reached 45°C. Next, the two mixtures were slowly combined, with mixing, and the combined mixtures were heated, with mixing, at 45°C for 30 minutes, at which time a solution was obtained. The solution was removed from the source of heat and allowed to cool gradually at room temperature.

Example 2

Another solution was prepared as described in Example 1, except that 3,0 grams of hydrocortisone was used and the amount of water was decreased by 0,5 grams.

Example 3

Another solution was prepared as described in Example 1, except that 50 grams of Polysorbate 20 and 47,5 grams of ethanol were used and no propylene glycol or water was used.

Example 4

Another solution was prepared as described in Example 1, except that 80 grams of Polysorbate 20 and 17,5 grams of ethanol were used, no propylene glycol or water was used, and the combined mixtures were heated overnight.

## Example 5

Another solution was prepared as described in Example 3, except that 47,5 grams of propylene glycol were used in place of the ethanol.

## Example 6

Another solution was prepared as described in Example 4, except that 17,5 grams of propylene glycol were used in place of the ethanol.

## Example 7

First, 2,5 grams of hydrocortisone was combined with 25,0 grams of ARLASOVE 200®, and the two ingredients were mixed with heating and stirring until the temperature reached 50°C and an even dispersion of the hydrocortisone in the surfactant was obtained. A second mixture was prepared by combining 35,0 grams of denatured ethanol, 20,5 grams of propylene glycol, 17 grams of water and 0,04 grams of benzalkonium chloride and then mixing the combined ingredients with heating until the temperature reached 50°C. Next, the two mixtures were slowly combined, with mixing, and the combined mixtures were heated, with mixing, at 50°C for 75 minutes, at which time a solution was obtained. The solution was removed from the source of heat and allowed to cool gradually at room temperature.

None of the foregoing description of the preferred embodiments is intended in any way to limit the scope of the invention which is set forth in the following claims. Those skilled in the art will recognize that many modifications, variations and adaptations are possible.

## Claims

**Claims for the following Contracting States : AT, BE, CH, DE, FR, GB, IT, LI, LU, NL, SE**

1. A solution of a corticosteroid comprising : at least 2,5% of the corticosteroid ; from at least 25% to 80% of a surfactant ; from 0 to 70% ethanol ; from 0 to 70% propylene glycol ; provided that the amount of ethanol or of propylene glycol or of the two together is at least 15% ; an effective amount of an antimicrobial agent ; and remainder water.

2. The solution of Claim 1 wherein the corticosteroid is hydrocortisone.

3. The solution of either Claim 1 or 2 wherein the surfactant is a nonionic surfactant.

4. The solution of Claim 1 wherein the ethanol is present at from at least 25% to 45%, the propylene glycol is present at from at least 10% to 30%, and the water is present at from at least 10% to 25.%.

5. The solution of Claim 4 wherein the corticosteroid is hydrocortisone.

6. The solution of either Claim 4 or Claim 5 wherein the surfactant is a nonionic surfactant.

7. The solution of Claim 6 wherein surfactant is Polysorbate 20.

8. The solution of Claim 7 wherein : the hydrocortisone is present at from at least 2,5% to 3%. ; the ethanol is denatured ethanol and is present at 35% ; the Polysorbate 20 is present at 25% ; the propylene glycol is present at 20,5% ; and the antimicrobial agent is benzalkonium chloride and is present at 0,04%.

9. The solution of Claim 6 wherein the surfactant is ARLASOVE 200®.

10. The solution of Claim 9 wherein : the hydrocortisone is present at from at least 2,5% to 3% ; the ethanol is denatured ethanol and is present at 35% ; the ARLASOVE 200® is present at 25% ; the propylene glycol is present at 20,5% ; and the antimicrobial agent is benzalkonium chloride and is present at 0,04%.

11. A method of making the solution of Claim 1 comprising : mixing the surfactant and the corticosteroid with heating until the temperature is from at least 5 to 10°C above the pour point of the surfactant; separately mixing the remaining ingredients with heating until the temperature is from at least 5 to 10°C above the pour point of the surfactant ; combining the two mixtures ; and heating the combined mixtures at a temperature from at least 5 to 10°C above the pour point of the surfactant with mixing until a solution is formed.

12. The method of Claim 11 wherein the corticosteroid is hydrocortisone.

13. The method of either Claim 11 or 12 wherein the surfactant is a nonionic surfactant.

14. The method of Claim 13 wherein the surfactant is Polysorbate 20.

15. The method of Claim 14 wherein the temperature is 45°C, and the combined mixtures are heated for 30 minutes.

16. The method of Claim 13 wherein the surfactant is ARLASOVE 200®.

17. The method of Claim 16 wherein the temperature is 50°C, and the combined mixtures are heated for 75 minutes.

**Claims for the following Contracting States : ES, GR**

1. A process for preparing a solution of a corticosteroid comprising at least 2,5% of the corticosteroid ; from at least 25% to 80% of a surfactant ; from 0 to 70% ethanol. ; from 0 to 70% propylene glycol ; provided that the amount of ethanol or of propylene glycol or of the two together is at least 15% ; an effective amount of an antimicrobial agent ; and remainder water by mixing the surfactant and the corticosteroid with heating until the temperature is from at least 5 to 10°C above the pour point of the surfactant ; separately mixing the remaining ingredients with heating until the temperature is from at least 5 to 10°C above the pour point of the surfactant ; combining the two mixtures ; and heating the combined mixtures at a temperature from at least 5 to 10°C above the pour point of the surfactant with mixing until a solution is formed.

2. A process according to claim 1 wherein the corticosteroid is hydrocortisone.

3. A process according of either claim 1 or 2 wherein the surfactant is a nonionic surfactant.

4. A process according to claim 3 wherein the surfactant is Polysorbate 20.

5. A process according to claim 4 wherein the temperature is 45 °C, and the combined mixtures are heated for 30 minutes

6. A process according to claim 3 wherein the surfactant is ARLASOVE 200®.

7. A process according to claim 6 wherein the temperature is 50°C, and the combined mixtures are heated for 75 minutes.

8. A process according to claim 1 wherein the ethanol is present at from at least 25% to 45%, the propylene glycol is present at from at least 10% to 30%, and the water is present at from at least 10% to 25.%.

9. A process according to claim 8 wherein the corticosteroid is hydrocortisone.

10. A process according of either claim 8 or claim 9 wherein the surfactant is a nonionic surfactant.

11. A process according to claim 10 wherein the surfactant is Polysorbate 20.

12. A process according to claim 11 wherein : the hydrocortisone is present at from at least 2,5% to 3%.; the ethanol is denatured ethanol and is present at 35% ; the Polysorbate 20 is present at 25% ; the propylene glycol is present at 20,5% ; and the antimicrobial agent is benzalkonium chloride and is present at 0,04%.

13. A process according to claim 10 wherein the surfactant is ARLASOVE 200®.

14. A process according to claim 13 wherein : the hydrocortisone is present at from at least 2,5% to 3%; the ethanol is denatured ethanol and is present at 35% ; the ARLASOVE 200® is present at 25% ; the propylene glycol is present at 20,5% ; and the antimicrobial agent is benzalkonium chloride and is present at 0,04%.

## Patentansprüche

### Patentansprüche für folgende Vertragsstaaten : AT, BE, CH, DE, FR, GB, IT, LI, LU, NL, SE

1. Lösung eines Corticosteroids, enthaltend : wenigstens 2,5% eines Corticosteroids, von wenigstens 25% bis zu 80% eines Tensids ; von 0 bis 70% Ethanol ; von 0 bis 70% Propylenglykol ; vorausgesetzt, daß die Menge des Ethanols oder des Propylenglykols oder beider zusammen wenigstens 15% beträgt ; eine effektive Menge eines antimikrobiell wirkenden Wirkstoffes ; und als Rest Wasser.

2. Lösung gemäß Anspruch 1, worin das Corticosteroid Hydrocortison ist.

3. Lösung gemäß Ansprüchen 1 oder 2, worin das Tensid ein nichtionisches Tensid ist.

4. Lösung gemäß Anspruch 1, worin das Ethanol von wenigstens 25% bis 45%, das Propylenglykol von wenigstens 10% bis 30% und das Wasser von wenigstens 10% bis 25% vorhanden sind.

5. Lösung gemäß Anspruch 4, worin das Corticosteroid Hydrocortison ist.

6. Lösung gemäß Ansprüchen 4 oder 5, worin das Tensid ein nichtionisches Tensid ist.

7. Lösung gemäß Anspruch 6, worin das Tensid Polysorbat 20 ist.

8. Lösung gemäß Anspruch 7, worin : das Hydrocortison von wenigstens 2,5% bis 3% vorhanden ist ; das Ethanol denaturiertes Ethanol ist und bis zu 35% vorhanden ist, das Polysorbat 20 bis zu 25% vorhanden ist, das Propylenglykol bis zu 20,5% vorhanden ist und der antimikrobiell wirkende Wirkstoff Benzalkoniumchlorid ist und bis zu 0,04% vorhanden ist.

9. Eine Lösung gemäß Anspruch 6, in der das Tensid ARLASOVE 200® ist.

10. Lösung gemäß Anspruch 9, worin : das Hydrocortison von wenigstens 2,5% bis 3% vorhanden ist, das Ethanol denaturiertes Ethanol ist und bis 35% vorhanden ist. ARLASOVE 200® bis zu 25% vorhanden ist, das Propylenglykol vorhanden ist bis zu 20,5% und der antimikrobiell wirkende Wirkstoff Benzalkoniumchlorid vorhanden ist in einer Menge bis zu 0,04%.

11. Verfahren zur Herstellung einer Lösung gemäß Anspruch 1, welches umfaßt : Mischen des Tensids und des Corticosteroides unter Erhitzen, bis die Temperatur von wenigstens 5° bis 10°C über dem Siedepunkt des Tensides ist ; getrenntes Mischen der übrigen Inhaltsstoffe unter Erhitzen, bis die Temperatur von wenigstens 5° bis 10°C oberhalb des Siedepunktes des Tensides ist. Vereinigen der beiden Mischungen und weiteres Erhitzen der verbundenen Mischungen bei einer Temperatur von wenigstens 5° bis 10°C oberhalb des Siedepunktes des Tensids unter Mischen, bis eine Lösung gebildet ist.

12. Verfahren gemäß Anspruch 11, worin das Corticosteroid Hydrocortison ist.

13. Verfahren nach Ansprüchen 11 oder 12, worin das Tensid ein nichtionisches Tensid ist.

14. Verfahren nach Anspruch 13, worin das Tensid Polysorbat 20 ist.

15. Verfahren gemäß Anspruch 14, worin die Temperatur 45°C und die vereinigten Mischungen für 30 Min. lang erhitzt werden.

16. Verfahren gemäß Anspruch 13, in dem das Tensid ARLASOVE 200® ist.

17. Verfahren gemäß Anspruch 16, worin die Temperatur 50°C beträgt und die vereinigten Mischungen für 75 Min. erhitzt werden.

## Patentansprüche für folgende Vertragsstaaten : ES, GR

1. Verfahren zur Herstellung einer Lösung eines Corticosteroides, das wenigstens 2,5% eines Corticosteroids umfaßt, von wenigstens 25% bis 80% eines Tensides, von 0 bis 70% Ethanol, von 0 bis 70% Propylenglykol, vorausgesetzt, daß die Menge des Ethanols oder des Propylenglykols oder beider zusammen wenigstens 15% beträgt, eine effektive Menge eines antimikrobiell wirkenden Wirkstoffes und ein Rest Wasser, indem das Tensid und das Corticosteroid unter Erhitzen gemischt werden, bis die Temperatur wenigstens 5 bis 10°C über dem Siedepunkt des Tensides liegt, getrenntes Mischen der übrigen Inhaltsstoffe unter Erhitzen bis die Temperatur wenigstens 5 bis 10°C über dem Siedepunkt des Tensides ist, Vereinigen der beiden Mischungen und anschließendes Erhitzen der Vereinigten Mischung bis. zu einer Temperatur von wenigstens 5 bis 10°C über dem Siedepunkt des Tensides unter weiterem Mischen, bis die Lösung gebildet ist.

2. Verfahren gemäß Anspruch 1, worin das Corticosteroid Hydrocortison ist.

3. Verfahren entsprechend Ansprüchen 1 oder 2, worin das Tensid ein nichtionisches Tensid ist.

4. Verfahren entsprechend Anspruch 3, worin das Tensid Polysorbat 20 ist.

5. Verfahren entsprechend Anspruch 4, worin die Temperatur 45°C beträgt und die vereinigten Mischungen für 30 Min. erhitzt werden.

6. Verfahren gemäß Anspruch 3, in dem das Tensid ARLASOVE 200® ist.

7. Verfahren gemäß Anspruch 6, worin die Temperatur 50°C beträgt und die vereinigten Mischungen für 75 Min. erhitzt werden.

8. Verfahren gemäß Anspruch 1, wonach Ethanol vorhanden ist, wenigstens 25% bis 45%, Propylenglykol von wenigstens 10% bis 30% vorhanden ist und Wasser von wenigstens 10% bis 25% vorhanden ist.

9. Verfahren gemäß Anspruch 8, worin das Corticosteroid Hydrocortison ist.

10. Verfahren gemäß Ansprüchen 8 oder 9, worin das Tensid ein nichtionisches Tensid ist.

11. Verfahren gemäß Anspruch 10, worin das Tensid Polysorbat 20 ist.

12. Verfahren gemäß Anspruch 11, worin : Hydrocortison vorhanden ist von wenigstens 2,5% bis 3%, das Ethanol denaturiertes Ethanol ist und bis zu 35% vorhanden ist, das Polysorbat 20 bis 25% vorhanden ist, das Propylenglykol bis zu 20,5% vorhanden ist und der antimikrobiell wirkende Wirkstoff Benzalkoniumchlorid ist und in einer Menge bis zu 0,04% vorhanden ist.

13. Verfahren gemäß Anspruch 10, worin das Tensid ARLASOVE 200® ist.

14. Verfahren gemäß Anspruch 13, worin : das Hydrocortison vorhanden ist von wenigstens 2,5% bis 3%, das Ethanol denaturiertes Ethanol ist und in einer Menge bis zu 35% vorhanden ist, das ARLASOVE 200® vorhanden ist bis zu 25%, das Propylenglykol bis zu 20,5% vorhanden ist und der antimikrobiell wirkende Wirkstoff Benzalkoniumchlorid ist und bis zu 0,04% vorhanden ist.

## Revendications

### Revendications pour les Etats contractants suivants : AT, BE, CH, DE, FR, GB, IT, LI, LU, NL, SE

1. Solution d'un corticostéroide contenant : au moins de 2,5% d'un corticostéroide ; au moins de 25% jusqu'à 80% d'un tensio-aktif ; de 0% jusqu'à 70% éthanol ; de 0% jusqu'à 70% propylène glycol ; supposé que la quantité d'éthanol ou de propylène glycol ou des deux en ensemble est au moins de 15%; une quantité effective d'un agent antimicrobien ; et un résidu d'eau.

2. Solution selon la revendication 1, caractérisé en ce que le corticostéroide est hydrocortisone.

3. Solution selon les revendications 1 ou 2 caractérisé en ce que le tensio-aktif est de manière non ionique.

4. Solution selon la revendication 1, caractérisé en ce que éthanol est présent au moins de 25% jusqu'à 45%, propylène glycol est présent au moins de 10% jusqu'à 30%, et d'eau est présente au moins de 10% jusqu'à 25%.

5. Solution selon la revendication 4, caractérisé en ce que le corticostéroide est hydrocortisone.

6. Solution selon les revendications 4 ou 5 caractérisé en ce que le tensio-aktif est un tensio-aktif non ionique.

7. Solution selon la revendication 6 caractérisé en ce que le tensio-aktif est Polysorbate 20.

8. Solution selon la revendication 7 caractérisé en ce que l'hydrocortisone est présent au moins de 2,5% jusqu'à 3% ; éthanol est éthanol dénaturé et présent à 35% ; le Polysorbate 20 est présent à 25%; le propylène glycol est présent à 20,5% ; et l'agent antimicrobien est le chlorure de benzalkonium et présent à 0,04%.

9. Solution selon la revendication 6 caractérisé en ce que le tensio-aktif est ARLASOVE 200®.

10. Solution selon la revendication 9 caractérisé en ce que l'hydrocortisone est présent au moins de 2,5% jusqu'à 3% ; éthanol est éthanol dénaturé et présent à 35% ; l'ARLASOVE 200® est présent à 25% ; le propylène glycol est présent à 20,5% ; et l'agent antimicrobien est le chlorure de benzalkonium et présent à 0,04%.

11. Procédé de préparation d'une solution selon la revendication 1 contenant : mêler le tensio-aktif et le corticostéroide sous chauffer jusqu'à la température a gagnée au moins de 5°C jusqu'à 10°C sur le point d'ébullition du tensio-aktif ; à part mêler des ingrédients restants sous chauffer jusqu'à la température a gagnée au moins de 5°C jusqu'à 10°C sur le point d'ébullition du tensio-aktif ; combiner des deux mélanges ; et chauffer les deux mélanges combinés à la température au moins de 5°C jusqu'à 10°C sur le point d'ébullition du tensio-aktif sous mêler jusqu'à une solution sera formée.

12. Procédé de préparation selon la revendication 11, caractérisé en ce que le corticostéroide est l'hydrocortisone.

13. Procédé de préparation selon les revendications 11 ou 12 caractérisé en ce que le tensio-aktif est un tensio-aktif non ionique.

14. Procédé de préparation selon la revendication 13 caractérisé en ce que le tensio-aktif est Polysorbate 20.

15. Procédé de préparation selon la revendication 14 caractérisé en ce que la température fait 45°C et les mélanges combinés sont chauffés pendant 30 minutes.

16. Procédé de préparation selon la revendication 13 caractérisé en ce que le tensio-aktif est ARLASOVE 200®.

17. Procédé de préparation selon la revendication 16 caractérisé en ce que la température fait 50°C et les mélanges combinés sont chauffés pendant 75 minutes.

## Revendications pour les Etats contractants suivants : ES, GR

1. Procédé de préparation d'une solution d'un corticostéroide contenant au moins de 2,5% de corticostéroide ; au moins de 25% jusqu'à 80% d'un tensio-aktif ; de 0% jusqu'à 70% éthanol ; de 0% jusqu'à 70% propylène glycol ; supposé que la quantité d'éthanol ou de propylène glycol ou des deux en ensemble est au moins de 15% ; une quantité effective d'un agent antimicrobien et un résidu d'eau tandis qu'en mêlant le tensio-aktif et le corticostéroide sous chauffer jusqu'à la température fait au moins de 5°C jusqu'à 10°C sur le point d'ébullition du tensio-aktif ; à part mêler des ingrédients restants sous chauffer jusqu'à la température a gagnée au moins de 5°C jusqu'à 10°C sur le point d'ébullition du tensio-aktif ; combiner des deux mélanges ; et chauffer les deux mélanges combinés à la température au moins de 5°C jusqu'à 10°C sur le point d'ébullition du tensio-aktif sous mêler jusqu'au moment une solution sera formée.

2. Procédé de préparation selon la revendication 1 caractérisé en ce que le corticostéroide est l'hydrocortisone.

3. Procédé de préparation selon les revendications 1 ou 2 caractérisé en ce que le tensio-aktif est un tensio-aktif non ionique.

4. Procédé de préparation selon la revendication 3 caractérisé en ce que le tensio-aktif est Polysorbate 20.

5. Procédé de préparation selon la revendication 4 caractérisé en ce que la température fait 45°C et les mélanges combinés sont chauffés pendant 30 minutes.

6. Procédé de préparation selon la revendication 3 caractérisé en ce que le tensio-aktif est ARLASOVE 200®.

7. Procédé de préparation selon la revendication 6 caractérisé en ce que la température fait 50°C et les mélanges combinés sont chauffés pendant 75 minutes.

8. Procédé de préparation selon la revendication 1 caractérisé en ce que l'éthanol est présent au moins de 25% jusqu'à 45%, le propylène glycol est présent au moins de 10% jusqu'à 30% et l'eau est présente au moins de 10% jusqu'à 25%.

9. Procédé de préparation selon la revendication 8 caractérisé en ce que le corticostéroide est l'hydrocortisone.

10. Procédé de préparation selon les revendications 8 ou 9 caractérisé en ce que le tensio-aktif est un tensio-aktif non ionique.

11. Procédé de préparation selon la revendication 10 caractérisé en ce que le tensio-aktif est Polysorbate 20.

12. Procédé de préparation selon la revendication 11 caractérisé en ce que l'hydrocortisone est présent au moins de 2,5% jusqu'à 3% ; l'éthanol est d'éthanol dénaturé et présent à 35 pour-cent ; le Polysorbate 20 est présent à 35% ; le propylène glycol est présent à 20,5 pour-cent ; et l'agent antimicrobien est le chlorure de benzalkonium et présent à 0,04%.

13. Procédé de préparation selon la revendication 10 caractérisé en ce que le tensio-aktif est ARLAOVE 200®.

14. Procédé de préparation selon la revendication 13 caractérisé en ce que l'hydrocortisone est présent au moins de 2,5% jusqu'à 3% ; l'éthanol est d'éthanol dénaturé et présent à 35% ; l'ARLASOVE 200® est présent à 25% ; le propylène glycol est présent à 20,5% ; et l'agent antimicrobien est le chlorure de benzalkonium et présent à 0,04%.